# EUROPÄISCHE PATENTSCHRIFT

(11) **EP 2 324 118 B1**
(45) Veröffentlichungstag und Bekanntmachung des Hinweises auf die Patenterteilung: **01.02.2012**
(21) Anmeldenummer: 09781863.7
(22) Anmeldetag: 14.08.2009
(51) Int. Cl.: C12P 3/00, C12N 1/00, C05F 7/00

(54) **Verfahren zur Phosphorgewinnung aus Feststoffen unter Verwendung laugungsaktiver und phosphatakkumulierender Mikroorganismen**
Process for the isolation of phosphorus from solid material employing leaching and phosphate-accumulating microorganisms
Procédé d'isolement de phosphore à partir d'un matériau solide en utilisant des microorganismes lixiviants et accumulants du phosphate

(30) Priorität: 15.08.2008 DE 102008038886
(43) Veröffentlichungstag der Anmeldung: 25.05.2011
(73) Patentinhaber: Georg Fritzmeier GmbH + Co. KG, 85655 Grosshelfendorf (DE)
(72) Erfinder: ZIMMERMANN, Jennifer, 52064 Aachen (DE); DOTT, Wolfgang, 52074 Aachen (DE)
(74) Vertreter: Winter, Brandl, Fürniss, Hübner, Röss, Kaiser, Polte - Partnerschaft
(86) Internationale Anmeldenummer: PCT/EP2009/060562
(87) Internationale Veröffentlichungsnummer: WO 2010/018228

(56) Entgegenhaltungen:
- FR-A1- 2 595 687
- HOLLENDER, J. ET AL.: "Selective enrichment and characterization of a phosphorus-removing bacterial consortium from activated sludge" APPLIED MICROBIOLOGY AND BIOTECHNOLOGY, Bd. 58, Nr. 1, Januar 2002 (2002-01), Seiten 106-111, XP002582231
- KREBS, W. ET AL.: "Growth stimulation of sulfur oxidizing bacteria for optimization of metal leaching efficiency of fly ash from municipal solid waste incineration" HYDROMETALLURGY, Bd. 59, Nr. 2-3, Februar 2001 (2001-02), Seiten 283-290, XP004227356
- TYAGI, R.D. ET AL.: "Bacterial leaching of metals from sewage sludge by indigenous iron-oxidizing bacteria" ENVIRONMENTAL POLLUTION, Bd. 82, Nr. 1, 1993, Seiten 9-12, XP002582232
- DATABASE WPI Week 200616 Thomson Scientific, London, GB; AN 2006-149331 XP002582233 & JP 2006 034141 A (UNIVERSITY OF HIROSHIMA) 9. Februar 2006 (2006-02-09)
- ZIMMERMANN, J. & DOTT, W.: "Sequenced bioleaching and bioaccumulation of phosphorus from sludge combustion - A new way of resource reclaiming" ADVANCED MATERIALS RESEARCH, Bd. 71-73, 2009, Seiten 625-628, XP002582234 DOI: 10.4028/www.scientific.net/AMR.71-73.625, online verfügbar seit dem 19. Mai 2009

## Beschreibung

Die Erfindung betrifft ein Verfahren zur selektiven Phosphorgewinnung aus schwermetall- und phosphathaltigem Feststoffmaterial.

Phosphor ist ein limitierender Nährstoff für das Wachstum von Pflanzen und wird, da er ausschließlich in gebundener Form vorkommt, als Phosphaterz in Lagerstätten abgebaut, die in der uns heute bekannten Form endlich sind. Abgebaute Phosphate werden hauptsächlich zu pflanzenverfügbarem Mineraldünger verarbeitet.

Bei der Behandlung von Abwässern aus Haushalten oder Industrie fällt Klärschlamm an, der aufgrund der darin enthaltenen Nährstoffe Stickstoff und Phosphor landwirtschaftlich verwertet werden kann. Da der Klärschlamm jedoch auch Schadstoffe wie Schwermetalle, beispielsweise Blei und Cadmium, enthält, wird die landwirtschaftliche Klärschlammverwertung zunehmend in Frage gestellt. Daher wird versucht, das im Klärschlamm enthaltene Phosphat durch gezielte Rückgewinnung möglichst weitgehend von Schwermetallen zu befreien.

Phosphor wird nur in ungelöster Form aus Abwasser entfernt oder eliminiert, wobei sich biologische und chemisch-physikalische Verfahren unterscheiden lassen.

Bei der chemisch-physikalischen Phosphorelimination erfolgt eine Fällung des gelösten Phosphats durch Zugabe von Fällmitteln. Als Fällmittel werden hauptsächlich Fe3⁺, Al³⁺, Fe²⁺ und Ca²⁺ verwendet. Nachteilig ist jedoch, dass die Fällmittel teilweise Neben- oder Abfallprodukte großtechnischer Prozesse sind und daher Verunreinigungen, wie z.B. Schwermetalle und organische Halogenverbindungen, enthalten, was die Schadstofffracht des Klärschlamms erhöht. Eisenphosphate können darüber hinaus nicht von Pflanzen aufgenommen werden. Der Einsatz reiner Fällmittel ist jedoch teuer.

Für eine adäquate Phosphorrückgewinnung aus Feststoffen wie Klärschlammasche ist es notwendig, chemisch gebundenen Phosphor in Lösung zu bringen. Die weitgehende Lösung von chemisch-physikalisch gebundenem Phosphor ist aber nur bei Verwendung von Säuren und bei niedrigen pH-Werten möglich. In der Regel erfolgt die Phosphatlösung über einen sauren Aufschluss durch Zugabe einer mineralischen Säure, gefolgt von selektiven Fällungsschritten. Im Seaborne-Verfahren findet beispielsweise zuerst eine anaerobe Schlammbehandlung statt, wobei der gebunden Phosphor im Anschluss durch Säurezugabe zusammen mit den Schwermetallen in Lösung gebracht wird. Anschließend wird der pH-Wert wieder angehoben, wobei die Schwermetalle mit H₂S selektiv gefällt und abgetrennt werden. In einem weiteren Schritt wird Phosphor durch Zugabe von zweiwertigen Metallen gezielt ausgefällt. Diese Verfahren sind aber zeitaufwändig und teuer.

Bei der vermehrten biologischen Phosphorelimination wird die Fähigkeit polyphosphatspeichernder oder -akkumulierender Mikroorganismen, insbesondere Bakterien, genutzt, Phosphor als energiereiches Polyphosphat in Form von Granula zu speichern. Dieses Verfahren ist als Bio-P-Verfahren bekannt und findet breite Anwendung in der Abwasserentsorgung zur Eliminierung von gelöstem Phosphat. Verfahren der vermehrten biologischen Phosphorelimination sind beispielsweise in DD 282 902-A5, DE-A-36 02 736 A1, DE 196 35 391 A1, GB 2 351 284 A und DE 10 2005 007 408 A1 beschrieben. Der durch biologische Phosphorelimination aus dem Abwasserstrom entfernte Phosphor wird dem System mit dem Überschussschlamm entnommen.

Die vermehrte biologische Phosphorelimination lässt sich jedoch nicht unmittelbar zur Rückgewinnung von chemisch gebundenem Phosphor verwenden. In phosphorhaltigen Feststoffen wie Klärschlammasche liegt Phosphor jedoch zumindest teilweise in chemisch gebundener Form vor. In Klärschlammaschen verbleibt Phosphor vollständig als Rückstand in der Asche. Obwohl Schwermetalle wie Pb, Cd, Cu, Cr, Hg, Ni und Zn in der Regel nur als Spurenkomponenten in der Asche vorliegen, kann es dennoch zu Überschreitungen der in der Düngemittelverordnung festgesetzten Grenzwerte kommen. Dies macht eine weitere Behandlung der Asche erforderlich. Außerdem ist im Boden die Verfügbarkeit des Phosphors, der in der Asche hauptsächlich als Apatit vorliegt, ohne weitere Behandlung für eine Nährstoffversorgung der Pflanzen nicht ausreichend.

Die Verfahren des Standes der Technik zur Rückgewinnung von Phosphor erfordern zahlreiche aufeinanderfolgende Fällungsschritte und eine exakte Dosierung der Fällungsmittel, was großtechnisch auf Grund schwankender Phosphormengen in den zu behandelnden Materialien technisch aufwändig ist. Das Fällen erfordert außerdem das Einbringen zusätzlicher chemischer Substanzen, was kostspielig ist und zu unerwünschten Umweltbelastungen führen kann.

Das Dokument HOLLENDER, J. ET AL.: "Selective enrichment and characterization of a phosphorus-removing bacterial consortium from activated sludge" (APPLIED MICROBIOLOGY AND BIOTECHNOLOGY, Bd. 58, Nr. 1, Januar 2002, Seiten 106-111) offenbart ein Verfahren zur selektiven Gewinnung von Phosphor aus phosphathaltigen Feststoffen, umfassend das Behandeln des phosphathaltigen Feststoffs mit polyphosphatspeichernden Mikroorganismen unter aeroben Bedingungen zur Aufnahme des Phosphats durch die polyphosphatspeichernden Mikroorganismen und das Abtrennen von mit Phosphor angereicherter Biomasse.

Das Dokument FR 2 595 687 A1 (SOCIÉTÉ CHIMIQUE DES CHARBONNAGES S.A., 18. September 1987) offenbart ein Verfahren zur Freisetzung von Phosphat aus phosphathaltigen Feststoffen, umfassend das Behandeln des phosphathaltigen Feststoffs mit laugungsaktiven Mikroorganismen unter sauren Bedingungen zum Freisetzen des Phosphats aus dem Feststoff, wobei das freigesetzte Phosphat zur Herstellung einer mit Phosphor angereicherten pflanzenverfügbaren Nährstoffquelle dient.

Die Dokumente KREBS, W. ET AL.: "Growth stimulation of sulfur oxidizing bacteria for optimization of metal leaching efficiency of fly ash from municipal solid waste incineration" (HYDROMETALLURGY, Bd. 59, Nr. 2-3, Februar 2001, Seiten 283-290) undTYAGI, R.D. ET AL.: "Bacterial leaching of metals from sewage sludge by indigenous iron-oxidizing bacteria" (ENVIRONMENTAL POLLUTION, Bd. 82, Nr. 1, 1993, Seiten 9-12) offenbaren Verfahren zur Freisetzung von Schwermetallen aus schwermetallhaltigen Feststoffen, umfassend das Behandeln des schwermetallhaltigen Feststoffs mit laugungsaktiven Mikroorganismen unter sauren Bedingungen zum Freisetzen der Schwermetalle aus dem Feststoff.

Aufgabe der vorliegenden Erfindung war es daher, ein einfaches und kostengünstiges Verfahren zur Gewinnung von Phosphor aus schwermetall- und phosphathaltigen Feststoffen bereitzustellen, bei dem der Phosphor frei von Schwermetallen isoliert werden kann. Es wurde nun gefunden, dass sich Phosphor effizient aus solchen Feststoffen Freisetzen und von Schwermetallen abtrennen lässt, wenn die Feststoffe einer gleichzeitigen Behandlung mit Laugungsaktiven, aeroben Schwefeloxidivenden Mikroorganismen und polyphosphat Speichenden Mikroorganismen unterzogen werden.

Gegenstand der vorliegenden Erfindung ist daher ein Verfahren zur selektiven Gewinnung von Phosphor aus schwermetall- und phosphathaltigen Feststoffen gemäß Anspruch 1, umfassend: , aerobe Schwefeloxidivende Mikroorganismen
- Behandeln des schwermetall- und phosphorhaltigen Feststoffs mit Mikroorganismen, die laugungsaktive und polyphosphatspeichernde Mikroorganismen umfassen, unter sauren aeroben Bedingungen zum Freisetzen von Schwermetallen und Phosphat aus dem Feststoff und zur Aufnahme des freigesetzten Phosphats durch die polyphosphatspeichernden Mikroorganismen; und
- Abtrennen von an Phosphor angereicherter Biomasse.

Überraschend wurde gefunden, dass polyphosphatspeichernde Mikroorganismen auch unter den sauren Bedingungen, unter denen eine Auslaugung von Schwermetallen und Phosphat erfolgt, zur Bioakkumulation von Phosphat in der Lage sind. Auf diese Weise lässt sich der Phosphor in Form von Biomasse von unerwünschten Schwermetallen abtrennen und einer weiteren Nutzung zuführen.

Laugungsaktive Mikroorganismen, wie sie erfindungsgemäß verwendet werden können, sind aerobe schwefeloxidierende Mikroorganismen, beispielsweise schwefeloxidierende Bakterien und Archaeen, wie sie bei bekannten herkömmlichen biologischen Laugungsverfahren ("Bioleaching") zur Gewinnung von Schwermetallen aus Erzen zur Anwendung kommen. Diese Mikroorganismen sind in der Lage, Schwermetallsulfide aufzulösen, indem Sulfide und elementarer Schwefel zu Sulfat oxidiert werden, wobei Schwefelsäure entsteht. Zum Laugen geeignete Mikroorganismen sind, ohne darauf beschränkt zu sein, Mikroorganismen der Gattungen Acidithiobacillus, Leptospirillum, Sulfobacillus, Acidimicrobium, Ferroplasma, Sulfolobus, Acidianus, Metallosphaerea, Fulvimonas, Rhodanobacter, Alicyclobacillus, Dyella, Dokdonella und Acidiphilium. Beispiele sind Mikroorganismen der Spezies *Acidithiobacillus thiooxidans, Acidithiobacillus ferrooxidans, Acidithiobacillus caldus, Acidithiobacillus albertensis, Leptospirillum ferrooxidans, Leptospirillum ferriphilum, Fulvimonas soli, Rhodanobacter thiooxydans, Alicyclobacillus ferrooxydans, Dyella yeojuensis, Dokdonella koreensis* und *Acidiphilum cryptum.* Acidithiobacillus Spezies wie *Acidithiobacillus thiooxidans* und *Acidithiobacillus ferrooxidans* sind besonders bevorzugte laugungsaktive Mikroorganismen.

Polyphosphatspeichernde Mikroorganismen, beispielsweise polyphosphatspeichernde Bakterien (auch als Bio-P-Bakterien bekannt), sind aerobe Mikroorganismen, die mehr Phosphor als üblich aufnehmen und in der Zelle speichern. Phosphatrefixierende und polyphosphatspeichernde Bakterien finden sich wie bekannt beispielsweise in Kläranlagen, beispielsweise im Bio-P-Becken und in anaerob stabilisiertem Klärschlamm (Faulschlamm), einer Mischung aus etwa 95 bis 99% Wasser und 5 bis 1% Feststoffen. Treten im belebten Schlamm eines Bio-P-Beckens oder wie im Faulturm anaerobe/anoxische Verhältnisse auf, so sind zahlreiche aerobe Mikroorganismen nicht mehr in der Lage, Nährstoffe aufzunehmen. Polyphosphatspeichernde Mikroorganismen nutzen unter diesen Stressbedingungen die Energie aus den gespeicherten Polyphosphaten unter Phosphatfreisetzung zur Nährstoffaufnahme. Steht den Bakterien anschließend wieder Sauerstoff zur Verfügung, füllen die Bakterien ihren Energiespeicher in Form von Polyphosphat wieder auf. Dabei wird mehr Phosphat aufgenommen, als zuvor abgegeben wurde. Phosphatspeichernde Mikroorganismen in einem Zustand nach anaeroben Stressbedingungen, die unter aeroben Bedingungen ihren Phosphatspeicher wieder auffüllen, werden nachfolgend auch als "anaerob konditionierte" polyphosphatspeichernde Mikroorganismen bezeichnet. "Anaerob konditionierte" Mikroorganismen werden bevorzugt verwendet. Beispielhafte polyphosphatspeichernde Mikroorganismen, die bei dem erfindungsgemäßen Verfahren zur Anwendung kommen können, sind, ohne darauf beschränkt zu sein, Mikroorganismen der Gattungen Pseudomonas, Aeromonas, Rhodocyclus, Tetrasphera und Acinetobacter.

Erfindungsgemäß werden laugungsaktive aerobe schwefeloxidierende Mikroorganismen und polyphosphatspeichernde Mikroorganismen zusammen als Laugungsflüssigkeit eingesetzt, wobei das unter den sauren Bedingungen des Verfahrens freigesetzte Phosphat von den phosphatrefixierenden und polyphosphatspeichernden Mikroorganismen akkumuliert wird. Die Mikroorganismen können aus Einzelkulturen oder beispielsweise aus Bodenproben oder Schlämmen stammen. Sie können allein oder zusammen in geeigneten Medien angezogen, im Falle der polyphosphatspeichernden Mikroorganismen gegebenenfalls zum Abbau der Phosphatspeicher anaerobem Stress ausgesetzt werden, und dann in Mischung unter sauren Bedingungen in dem erfindungsgemäßen Verfahren eingesetzt werden. Gewöhnlich enthalten die in dem erfindungsgemäßen Verfahren eingesetzten Mikroorganismen verschiedene Arten von laugungsaktiven und polyphosphatspeichernden Mikroorganismen. Je nach dem Ausgangsmaterial für die Mikroorganismen kann die erfindungsgemäß verwendete Mischung daher auch andere aerobe und anaerobe Mikroorganismen enthalten.

Bevorzugt wird die in dem erfindungsgemäßen Verfahren eingesetzte Mischung von Mikroorganismen jedoch erhalten, indem laugungsaktive schwefeloxidierende Mikroorganismen in einem wässrigen Ausgangsmaterial angereichert werden, das anaerob konditionierte polyphosphatspeichernde Mikroorganismen enthält. Beispiele für Ausgangsmaterialien, die polyphosphatspeichernde Mikroorganismen enthalten, sind anaerob stabilisierter Klärschlamm oder Material wie es sich in der anaeroben Stufe eines Bio-P-Beckens findet. Anaerob stabilisierter Klärschlamm wird als Ausgangsmaterial bevorzugt. Die Anreicherung der schwefeloxidierenden Mikroorganismen erfolgt zweckmäßig, indem man ein solches Ausgangsmaterial, das üblicherweise als endogene Mikroorganismen auch schwefeloxidierende Mikroorganismen enthält, insbesondere *Acidithiobacillus thiooxidans* und *Acidithiobacillus ferrooxidans,* unter Zugabe einer oxidierbaren Schwefelquelle, beispielsweise in Form von elementarem Schwefel oder Sulfiden, bevorzugt zusammen mit Eisensulfat (FeS04), unter aeroben Bedingungen kultiviert. Schwefeloxidierende Mikroorganismen verwenden CO₂ als Kohlenstoffquelle und wachsen unter diesen Bedingungen bevorzugt. Gegebenenfalls können dem Ausgangsmaterial gewünschte schwefeloxidierende Mikroorganismen aber auch zugegeben werden, die dann angereichert werden. Die Kultivierung erfolgt vorzugsweise bei einer Temperatur zwischen 15 und 37°C, bevorzugt zwischen 20 und 30°C. Da schwefeloxidierende Mikroorganismen wie *Acidithiobacillus thiooxidans* hierbei in großer Menge Schwefelsäure produzieren, kann der pH-Wert des Ausgangsmaterials, der üblicherweise zwischen 7 und 8 liegt, bis auf unter 2 sinken. Es wurde überraschend gefunden, dass die polyphosphatspeichernden Mikroorganismen eine Kultivierung unter diesen sauren Bedingungen tolerieren. Die Kultivierung wird so lange fortgesetzt, bis der pH den gewünschten Wert erreicht hat, vorteilhaft einen pH-Wert von 4,0 oder niedriger, besonders bevorzugt zwischen 1,0 und 3,5, beispielsweise zwischen 1,5 und 3,5 oder 2,0 und 3,5, und ganz besonders bevorzugt zwischen 1,5 und 2,5 beispielsweise zwischen 2,2 und 2,5. Danach werden die Feststoffteilchen bevorzugt abgetrennt. Die bei der Kultivierung erhaltene Kulturflüssigkeit, die die schwefeloxidierenden und polyphosphatspeichernden Mikroorganismen enthält, kann als Laugungsflüssigkeit in dem erfindungsgemäßen Verfahren eingesetzt werden. Eine solche Laugungsflüssigkeit kann dementsprechend auch noch andere aerobe oder anaerobe Mikroorganismen enthalten, die in dem Ausgangsmaterial vorhanden sind.

Die mikrobielle Zusammensetzung, die auf diese Weise erhalten wird und angereicherte laugungsaktive, aerobe schwefeloxidierende und anaerob konditionierte polyphosphatspeichernde Mikroorganismen enthält, ist ebenfalls Gegenstand der Erfindung.

Feststoffe, die nach dem erfindungsgemäßen Verfahren zur Phosphatgewinnung behandelt werden können, sind laugbare schwermetall- und phosphathaltige Feststoffe. Solche Feststoffe können natürlich vorkommen oder nach Wärmebehandlung oder Wasserentzug erhalten werden. Beispiele für Feststoffe, die mit dem erfindungsgemäßen Verfahren vorteilhaft behandelt werden können, sind Verbrennungsaschen wie Klärschlammasche, Tier- und Knochenmehl, Industrieschlacke, Bodenmaterial, Schlämme, Deponieböden und Gülle.

Bei der erfindungsgemäßen Behandlung der Feststoffe oxidieren die laugungsaktiven Mikroorganismen die Schwermetallsulfide und bringen somit die Metalle in Lösung. Parallel wird unter diesen sauren Laugungsbedingungen chemisch gebundener Phosphor aufgeschlossen und ebenfalls in Lösung gebracht. Falls der Schwefelgehalt der behandelten Feststoffe sehr gering ist, kann die Behandlung auch unter Zugabe von oxidierbarem Schwefel erfolgen, beispielsweise in Form von elementarem Schwefel oder Sulfiden.

Das erfindungsgemäße Verfahren wird üblicherweise bei einem pH-Wert von ≤ 4,0, besonders bevorzugt zwischen 1,0 und 3,5, beispielsweise zwischen 1,5 und 3,5 oder 2,0 und 3,5 und ganz besonders bevorzugt zwischen 1,5 und 2,5, beispielsweise zwischen 2,2 und 2,5 durchgeführt. Der optimale pH-Wert lässt sich vom Fachmann durch einfache Versuche ermitteln und gegebenenfalls stabil halten.

Das erfindungsgemäße Verfahren wird üblicherweise bei einer Temperatur zwischen 15 und 30°C und bevorzugt zwischen 20 und 25°C durchgeführt.

Aufgrund der überraschenden Tolerierung der niedrigen pH-Werte durch die phosphatrefixierenden und polyphosphatspeichernden Bakterien, sind sie immer noch in der Lage, freigesetztes Phosphat verstärkt zu akkumulieren. Dies kann sehr schnell geschehen, da für die Aerobier ausreichend Sauerstoff als Elektronenakzeptor vorliegt. Unter diesen Bedingungen kommt es zu einem expotentiellen Wachstum der phosphatrefixierenden und polyphosphatspeichernden Bakterien, und das gelaugte Phosphat wird refixiert oder als endogener Energieträger Polyphosphat akkumuliert. Die gelaugten Schwermetalle verbleiben in Lösung.

Üblicherweise erfolgt die Behandlung der schwermetall- und phosphorhaltigen Feststoffe durch Perkolation von saurer Laugungsflüssigkeit, die laugungsaktive, aerobe schwefeloxidierende und anaerob konditionierte polyphosphatspeichernde Mikroorganismen enthält, durch das zu behandelnde Medium. Diese Behandlung kann beispielsweise als Halden- oder Haufenlaugung oder als Mietenperkolation (Feuchtmiete) erfolgen. Vorteilhaft befindet sich der zu laugende Feststoff in einem Perkolator, durch den saure Laugungsflüssigkeit perkoliert wird, die die laugungsaktiven, aeroben schwefeloxidierenden und die anaerob konditionierten polyphosphatspeichernden Mikroorganismen enthält. Die Laugungsflüssigkeit befindet sich in einem Vorratsbehälter, zweckmäßig einem Rührreaktor. Bevorzugt wird die perkolierte Laugungsflüssigkeit in den Perkolator zurückgeführt, vorzugsweise über den Vorratsbehälter. Durch diese Verfahrensweise wird die Auslaugungsrate verbessert. Durch die kontinuierliche Kreisführung wird außerdem ein optimales Lösungsverhältnis für die Schwermetalle und den Phosphor sowie eine kontinuierliche Zuführung von Mikroorganismen sichergestellt.

Die so entstandene mit Phosphat angereicherte Biomasse wird aus der Laugungslösung abgetrennt, beispielsweise durch Zentrifugation oder Filtration. Abgetrennte Mikroorganismen können kontinuierlich durch frische Mikroorganismen ersetzt werden. Zweckmäßig erfolgt die Abtrennung der mit Phosphor angereicherten Biomasse, wenn die Phosphatkonzentration in der Laugungsflüssigkeit ein Minimum erreicht hat. Neben der Biomasse können gegebenenfalls auch die mit Schwermetallen angereicherte Laugungsflüssigkeit und/oder die an Schwermetallen abgereicherten Feststoffmaterialien abgetrennt und isoliert werden und einer Wiederverwertung zugeführt werden.

Der akkumulierte Phosphor ist pflanzenverfügbar und frei von unerwünschten Schwermetallen. Die erhaltene Biomasse kann als pflanzenverfügbare Nährstoffquelle, beispielsweise als Biodünger, oder zur Bodenverbesserung verwendet werden. Das oben beschriebene Verfahren ermöglicht somit eine Biolaugung von metallhaltigen und phosphorreichen Feststoffen und die gleichzeitige selektive Gewinnung von Phosphor. Das erfindungsgemäße Verfahren ist daher eine kostengünstige und umweltfreundliche Recyclingmethode für den Nährstoff Phosphor. Das Verfahren eignet sich gleichzeitig hervorragend zur Dekontamination von schadstoffhaltigen Feststoffen, die von Schwermetallen abgereichert werden, und damit zur Bodensanierung. Die an Schwermetall abgereicherten Feststoffe können problemlos wieder verwertet werden, beispielsweise als Baustoffe, insbesondere im Straßenbau, da die für solche Verwendungen erforderlichen Grenzwerte der Schwermetallbelastung nicht überschritten werden. Darüber hinaus kann das erfindungsgemäße Verfahren auch zur gleichzeitigen effizienten Mobilisierung und Wiedergewinnung von Schwermetallen aus den behandelten Feststoffmaterialien eingesetzt werden, indem die Schwermetalle, die in der resultierenden Laugungslösung enthalten sind, beispielsweise über Membrananlagen aufkonzentriert werden.

Die vorliegende Erfindung wird durch das nachfolgende Ausführungsbeispiel unter Bezug auf die beiliegenden Figuren näher erläutert.
- Figur 1: zeigt eine schematische Darstellung des erfindungsgemäßen Verfahrens.
- Figur 2: zeigt die Auslaugungsrate von Metallen aus Klärschlammasche bei Verwendung von mit Acidithiobacillus angereichertem Klärschlamm (AEDS) nach 11 Tagen Behandlung. Die_Angaben_beziehen sich auf den Gesamtgehalt der Metalle in der Klärschlammasche.
- Figur 3: zeigt die gelöste Phosphatmenge bei AEDS (Kreise) im Vergleich mit einer reinen Acidithiobacillus-Kultur (Quadrate) über einen Zeitraum von 11 Tagen.

### Ausführungsbeispiel

Als Ausgangsmaterial wurde anaerob konditionierter Klärschlamm (Faulschlamm, Feststoffgehalt ca. 6 %) eingesetzt, der aus einer städtischen Kläranlage erhalten wurde. Als Folge einer gewissen Verweildauer in einem Faulturm enthält dieser Faulschlamm anaerob konditionierte polyphosphatspeichernde Mikroorganismen mit leeren Phosphatspeichern. Die Proben wurden in 21-Polypropylen-Flaschen gesammelt und bis zur Nutzung bei 4°C gelagert.

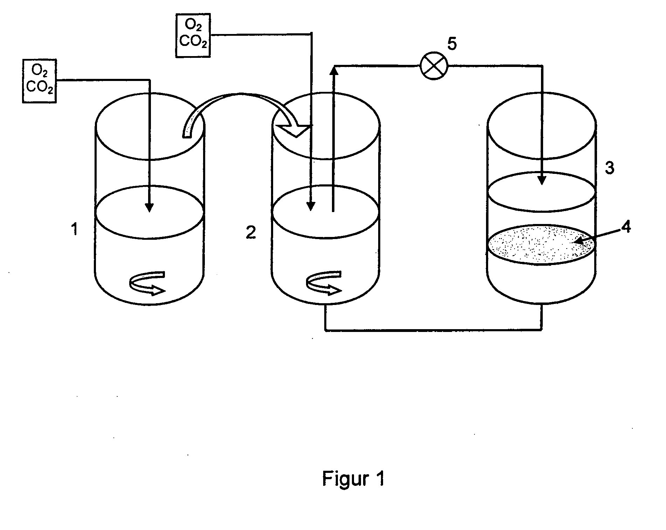

Laugungsaktive, aerobe schwefeloxidierende Mikroorganismen wurden in dem Faulschlamm unter Zugabe von elementarem Schwefel als Energiequelle und Zufuhr von CO₂ als Kohlenstoffquelle angereichert. Gemäß Figur 1 wurden 2 I anaerob konditionierter Klärschlamm in einem 21-Rührreaktor (1) unter Rühren (250 Upm) und Belüftung mit sauerstoff- und kohlendioxidhaltiger Druckluft mit 10 g/l elementarem Schwefel versetzt. Die Anreicherung fand bei Raumtemperatur (25°C) ohne Zugabe von weiteren Nährstoffen statt. Zur Kontrolle des Bakterienwachstums wurde der pH-Wert täglich gemessen. Nach Erreichen eines pH-Werts von 2,3 bis 2,4 (nach ca. 22 Tagen), wurde der Ansatz bei 25000 g für 20 min zentrifugiert. Der mit laugungsakeriven, aeroben schwefeloxidierenden Mikroorganismen angereicherte und nach wie vor polyphosphatspeichernde Mikroorganismen enthaltende Überstand (ca. 800 ml, im Folgenden als AEDS bezeichnet) wurde in einen zweiten Rührreaktor (2) überführt und als Laugungslösung verwendet. Der Rückstand wurde verworfen.

Der Rührreaktor (2) ist mit einem Perkolator (3) mit Glasfritte (4) verbunden, worauf sich der zu laugende Feststoff befindet. In dem hier beschriebenen Beispiel wurden 2 g Klärschlammasche verwendet.

Mit einer Schlauchpumpe (5) (Flussrate 25 ml/min) wurde_der_AEDS aus dem Rührreaktor (2) in den Perkolator (3) und über Glasfritte (4) mit dem zu laugenden Feststoff und wieder zurück in den Reaktor (2) geführt, wobei dieser Kreislauf 11 Tage aufrechterhalten wurde. Der pH-Wert blieb bei dieser Vorgehensweise im Wesentlichen stabil. Alle 24 h erfolgte eine Probenentnahme (ca. 7 ml), wobei die Proben mit einem 0,45 µm Filter filtriert wurden.

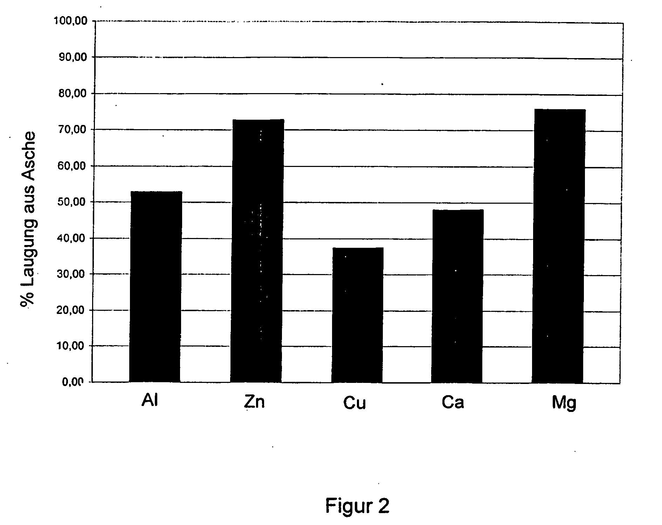

Proben für die Schwermetallanalytik wurden mit konz. HNO₃ versetzt (Verhältnis 1:3). Der Anteil der durch den AEDS freigesetzten Schwermetalle wurden nach 11 Tagen Behandlung mit ICP-MS (Massenspektrometrie mit induktiv gekoppeltem Plasma) bestimmt. In Figur 2 ist der Gehalt einiger Schwermetalle in Lösung bezogen auf den Gesamtgehalt der Schwermetalle in der Klärschlammasche dargestellt. Demnach gingen zwischen 40% (Kupfer) und 70% (Zink) der in der Klärschlammasche enthaltenen Schwermetalle in Lösung.

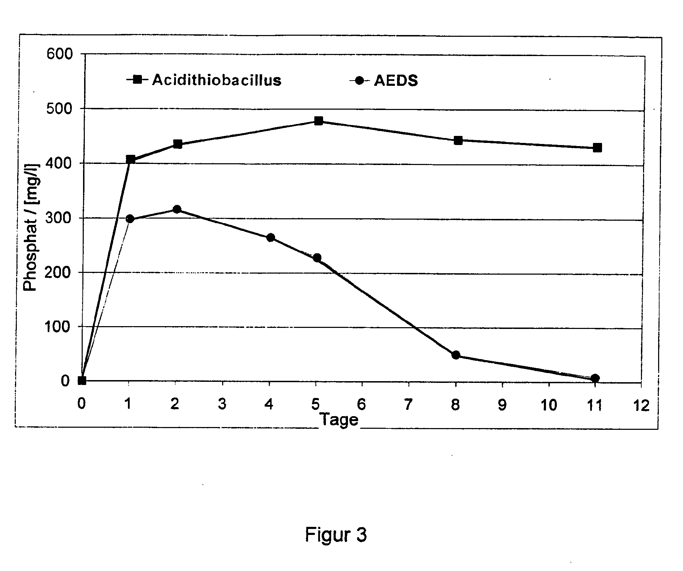

Der Phosphorgehalt des AEDS wurde mittels Ionenchromatographie und photometrischer Messungen bestimmt. Das gelöste Phosphat bzw. die Phosphatakkumulation durch von in AEDS enthaltenen phosphatakkumulierenden Bakterien im zeitlichen Verlauf ist in Figur 3 dargestellt (Kreise). Etwa 2-4 Tage nach Behandeln der Klärschlammasche mit AEDS wurde eine deutliche Eintrübung des AEDS beobachtet, die durch das Wachstum der in der Laugungslösung vorhandenen phosphatakkumulierenden Bakterien verursacht wurde. Dies war auch der Zeitpunkt, ab dem die Bioakkumulation des durch die Biolaugung freigesetzten Phosphors deutlich zunahm. Wie Figur 3 zu entnehmen ist, sank durch die Akkumulation die Menge an gelöstem Phosphat von ca. 300 mg/l an Tag 3 auf annähernd 0 mg/ml an Tag 11. Wurde hingegen eine Reinkultur von Acidithiobacillus in einem eigens für sie optimierten Nährmedium verwendet, in der keine phosphatakkumulierenden Bakterien enthalten waren, blieb die Phosphorakkumulation aus der Lösung weitgehend aus (Figur 3, Quadrate).

Die phosphatreiche Biomasse wurde durch Zentrifugation von den weiterhin gelösten Schwermetallen abgetrennt, wenn der Phosphorgehalt in der Lösung einen minimalen Wert erreicht hatte.

Das erfindungsgemäße Verfahren ermöglicht somit eine effiziente Biolaugung von metallhaltigen und phosphorreichen Feststoffen bei gleichzeitiger selektiver Gewinnung von Phosphor. Die mobilisierten Schwermetalle können ebenfalls aus den behandelten Feststoffen zurückgewonnen werden.

## Patentansprüche

1. Verfahren zur selektiven Gewinnung von Phosphor aus schwermetall- und phosphathaltigen Feststoffen, umfassend:
- Behandeln des schwermetall- und phosphorhaltigen Feststoffs mit Mikroorganismen, die laugungsaktive, aerobe schwefeloxidierende Mikroorganismen und polyphosphatspeichernde Mikroorganismen umfassen, unter sauren aeroben Bedingungen zum Freisetzen von Schwermetallen und Phosphat aus dem Feststoff und zur Aufnahme des freigesetzten Phosphats durch die polyphosphatspeichernden Mikroorganismen; und
- Abtrennen von mit Phosphor angereicherter Biomasse.

2. Verfahren nach Anspruch 1, wobei die polyphosphatspeichernden Mikroorganismen anaerob konditionierte polyphosphatspeichernde Mikroorganismen sind.

3. Verfahren nach Anspruch 1 oder 2, wobei der behandelte Feststoff Klärschlammasche, Industrieschlacke, Bodenmaterial, Schlämme, Deponieböden oder Gülle ist.

4. Verfahren nach einem der Ansprüche 1 bis 3, wobei die Behandlung des Feststoffs bei einem pH-Wert zwischen 2,0 und 3,5 durchgeführt wird.

5. Verfahren nach einem der Ansprüche 1 bis 4, wobei die Behandlung des Feststoffs bei einer Temperatur zwischen 15 und 37°C durchgeführt wird.

6. Verfahren nach einem der Ansprüche 1 bis 5, wobei die Behandlung unter Zugabe von oxidierbarem Schwefel erfolgt.

7. Verfahren nach einem der Ansprüche 1 bis 6, umfassend die Schritte:
(a) Anreicherung von laugungsaktiven, aeroben schwefeloxidierenden Mikroorganismen durch Kultivierung in einem wässrigen Ausgangsmaterial, das anaerob konditionierte polyphosphatspeichernde Mikroorganismen enthält;
(b) Behandeln des schwermetall- und phosphorhaltigen Feststoffs mit der in Schritt (a) erhaltenen sauren Laugungsflüssigkeit unter aeroben Bedingungen zum Freisetzen von Schwermetallen und Phosphat aus dem Feststoff und zur Aufnahme des freigesetzten Phosphats durch die in der Kultur enthaltenen polyphosphatspeichernden Mikroorganismen; und
(c) Abtrennen von mit Phosphor angereicherter Biomasse.

8. Verfahren nach Anspruch 7, wobei das Ausgangsmaterial anaerob konditionierter Klärschlamm ist.

9. Verfahren nach einem der Ansprüche 1 bis 8, wobei sich der Feststoff in einem Perkolator befindet, durch den saure Laugungsflüssigkeit perkoliert, die laugungsaktive, aerobe schwefeloxidierende Mikroorganismen und anaerob konditionierte polyphosphatspeichernde Mikroorganismen enthält.

10. Verfahren nach Anspruch 9, wobei die perkolierte Laugungsflüssigkeit kontinuierlich in den Perkolator zurückgeführt wird.

11. Verfahren nach einem der Ansprüche 1 bis 10, wobei die Behandlung unter Zuführung frischer laugungsaktiver, aerober schwefeloxidierender Mikroorganismen und polyphosphatspeichernder Mikroorganismen durchgeführt wird.

12. Verfahren nach einem der Ansprüche 1 bis 11, wobei die Abtrennung der mit Phosphor angereicherten Biomasse erfolgt, wenn die Phosphatkonzentration in der Laugungsflüssigkeit ein Minimum erreicht hat.

13. Verfahren nach einem der Ansprüche 1 bis 12, wobei neben der mit Phosphor angereicherten Biomasse die mit Schwermetallen angereicherte Laugungsflüssigkeit und/oder das an Schwermetallen abgereicherten Feststoffmaterial isoliert werden.

14. Mikrobielle Zusammensetzung, erhältlich durch Anreicherung von laugungsaktiven, aeroben schwefeloxidierenden Mikroorganismen in einem wässrigen Ausgangsmaterial, das anaerob konditionierte polyphosphatspeichernde Mikroorganismen enthält.

## Claims

1. A method of selectively obtaining phosphorus from solids containing heavy metals and phosphates, including:
- treating the solid containing heavy metals and phosphorus with microorganisms which include aerobic sulfur-oxidizing microorganisms presenting leaching activity and polyphosphate-storing microorganisms, under acidic aerobic conditions, for the liberation of heavy metals and phosphate from the solid and for assimilation of the liberated phosphate by the polyphosphate-storing microorganisms; and
- separating phosphorus-enriched biomass.

2. The method according to claim 1, wherein the polyphosphate-storing microorganisms are anaerobically conditioned, polyphosphate-storing microorganisms.

3. The method according to claim 1 or 2, wherein the treated solid is sewage sludge ashes, industrial slags, soil material, sludges, landfills, or liquid manure.

4. The method according to any one of claims 1 to 3, wherein the treatment of the solid is carried out at a pH between 2.0 and 3.5.

5. The method according to any one of claims 1 to 4, wherein the treatment of the solid is carried out at a temperature between 15 and 37°C.

6. The method according to any one of claims 1 to 5, wherein the treatment takes place under addition of oxidizable sulfur.

7. The method according to any one of claims 1 to 6, including the steps of:
(a) enriching aerobic sulfur-oxidizing microorganisms presenting leaching activity by cultivation in an aqueous starting material which contains anaerobically conditioned, polyphosphate-storing microorganisms;
(b) treating the solid containing heavy metals and phosphorus with the acidic leaching liquid obtained in step (a), under aerobic conditions, for the liberation of heavy metals and phosphate from the solid and for assimilation of the liberated phosphate by the polyphosphate-storing microorganisms contained in the culture; and
(c) separating biomass enriched with phosphorus.

8. The method according to claim 7, wherein the starting material is anaerobically conditioned sewage sludge.

9. The method according to any one of claims 1 to 8, wherein the solid is present in a percolator through which acidic leaching liquid containing aerobic sulfur-oxidizing microorganisms presenting leaching activity and anaerobically conditioned, polyphosphate-storing microorganisms percolates.

10. The method according to claim 9, wherein the percolated leaching liquid is continuously recirculated into the percolator.

11. The method according to any one of claims 1 to 10, wherein the treatment is carried out while supplying fresh aerobic sulfur-oxidizing microorganisms and polyphosphate-storing microorganisms presenting leaching activity.

12. The method according to any one of claims 1 to 11, wherein the separation of the biomass enriched with phosphorus takes place when the phosphate concentration in the leaching liquid has reached a minimum.

13. The method according to any one of claims 1 to 12, wherein in addition to the biomass enriched with phosphorus, the leaching liquid enriched with heavy metals and/or the solid material depleted in heavy metals is/are isolated.

14. A microbial composition obtainable through enriching aerobic sulfur-oxidizing microorganisms presenting leaching activity in an aqueous starting material which contains anaerobically conditioned, polyphosphate-storing microorganisms.

## Revendications

1. Procédé d'isolement sélectif de phosphore à partir de solides contenant des métaux lourds et des phosphates, comprenant
- le traitement du solide contenant des métaux lourds et du phosphore avec des micro-organismes qui comprennent des micro-organismes lixiviants, aérobies, oxydant le soufre et des microorganismes accumulant les polyphosphates, dans des conditions aérobies acides pour libérer les métaux lourds et le phosphate du solide et pour absorber le phosphate libéré par les micro-organismes accumulant les polyphosphates ; et
- la séparation de la biomasse enrichie en phosphore.

2. Procédé selon la revendication 1, dans lequel les micro-organismes accumulant les polyphosphates sont des micro-organismes accumulant les polyphosphates conditionnés de façon anaérobie.

3. Procédé selon la revendication 1 ou 2, dans lequel le solide traité est constitué par des cendres de boues d'épuration, des laques industrielles, des matériaux de sol, des boues, des sols de décharge ou des lisiers.

4. Procédé selon l'une des revendications 1 à 3, dans lequel le traitement du solide est réalisé à un pH entre 2,0 et 3,5.

5. Procédé selon l'une des revendications 1 à 4, dans lequel le traitement du solide est réalisé à une température entre 15 et 37° C.

6. Procédé selon l'une des revendications 1 à 5, dans lequel le traitement s'effectue en ajoutant du soufre oxydable.

7. Procédé selon l'une des revendications 1 à 6, comprenant les étapes suivantes :
(a) enrichissement de micro-organismes lixiviants, aérobies, oxydant le soufre par culture dans un matériau de départ aqueux qui contient des micro-organismes accumulant des polyphosphates conditionnés de façon anaérobie ;
(b) traitement du solide contenant des métaux lourds et du phosphore avec le fluide lixiviant acide obtenu à l'étape (a) dans des conditions aérobies pour libérer les métaux lourds et le phosphate du solide et pour absorber le phosphate libéré par les micro-organismes accumulant les polyphosphates contenus dans la culture ; et
(c) séparation de la biomasse enrichie en phosphore.

8. Procédé selon la revendication 7, dans lequel le matériau de départ est une boue d'épuration conditionnée de façon anaérobie.

9. Procédé selon l'une des revendications 1 à 8, dans lequel le solide se trouve dans un percolateur dans lequel le fluide de lixivation est percolé, qui contient des micro-organismes lixiviants, aérobies, oxydant le soufre et des micro-organismes accumulant les polyphosphates conditionnés de façon anaérobie.

10. Procédé selon la revendication 9, dans lequel le fluide lixiviant percolé est acheminé en continu dans le percolateur.

11. Procédé selon l'une des revendications 1 à 10, dans lequel le traitement est réalisé en acheminant des micro-organismes lixiviants frais, aérobies, oxydant le soufre et des micro-organismes accumulant les polyphosphates.

12. Procédé selon l'une des revendications 1 à 11, dans lequel la séparation de la biomasse enrichie en phosphore s'effectue lorsque la concentration en phosphate a atteint un minimum dans le fluide de lixivation.

13. Procédé selon l'une des revendications 1 à 12, dans lequel, parallèlement à la biomasse enrichie en phosphore, le fluide de lixiviation enrichi en métaux lourds et/ou le matériau solide appauvri en métaux lourds sont isolés.

14. Composition microbienne pouvant être obtenue par enrichissement de micro-organismes lixiviants, aérobies, oxydant le soufre dans un matériau de départ aqueux qui contient des micro-organismes accumulant les polyphosphates conditionnés de façon anaérobie.
